# EUROPEAN PATENT SPECIFICATION

(11) **EP 0 984 916 B1**
(45) Date of publication and mention of the grant of the patent: **26.06.2002**
(21) Application number: 98928285.0
(22) Date of filing: 16.05.1998
(51) Int. Cl.: C07C 59/305, C07C 69/708

(54) **OLIGOMERS OF FATTY ACIDS**
FETTSÄUREN-OLIGOMERE
OLIGOMERES D'ACIDES GRAS

(30) Priority: 27.05.1997 EP 97201603
(43) Date of publication of application: 15.03.2000
(73) Proprietor: UNICHEMA CHEMIE B.V., 2802 BE Gouda (NL)
(72) Inventor: OOSTWAL, Michel, Rua Marecha Hastim philo, CEP-05641-900 S o Paulo, SP (BR); MINNEE, Jacobus, Samuel, NL-2741 KX Waddinxveen (NL); MULDER, Geertroi, Pietertje, NL-2411 ZA Bodegraven (NL); DE MOOY, Abraham, Cornelis, NL-2802 RS Gouda (NL)
(74) Representative: Humphries, Martyn
(86) International application number: EP9802900
(87) International publication number: WO9854120

(56) References cited:
- DE-A- 4 038 608
- DE-A- 4 115 146
- DE-C- 4 125 031
- JOHNSON P Y ET AL: "THE REFORMATSKY REACTION OF ETHYL ALPHA-BROMO ESTERS WITH BIS(CHLOROMETHYL) ETHER" JOURNAL OF ORGANIC CHEMISTRY, vol. 38, no. 13, 29 June 1973, pages 2346-2350, XP002035962

## Description

The present invention relates to compositions comprising dihydroxy ether oligomers of fatty acids and/or esters thereof, a process for manufacturing the compositions, and their use as building blocks in subsequent further processing for use for polymers, paints, inks, etc.

Dihydroxy ether oligomers of fatty acids and/or esters thereof are herein to be understood to comprise such dimers of fatty acids and/or esters thereof, as well as higher such oligomers thereof, such as the trimers and tetramers. Fatty acids are herein to be understood to comprise both fatty acids as well as fatty acid esters, like triglycerides or lower alkyl or alkenyl esters e.g. with 1-6 carbon atoms.

In one aspect, the present invention relates to compositions comprising dihydroxy ether dimers of fatty acids, a process for manufacturing the compositions, and their use as building blocks in subsequent further processing for use for polymers, paints, inks, etc.

A well-known group of building blocks for e.g. polyurethanes and polyesters are dimerised fatty acids. These dimers are predominantly straight chain dicarboxylic fatty acids, having at both ends a carboxylic group, and are obtainable by subjecting unsaturated fatty acids such as oleic acid to an acid-catalysed high temperature treatment.

Such dimers are e.g. PRIPOL 1004 and 1006, as marketed by Uniqema.

Although simple fatty acid dimers are suitable for many applications, there has been a demand for such long dimers having additional functional groups besides the two carboxylic groups present.

This would enable a wider field of applications, and may even lead to new applications and materials.

For some purposes, it may be preferred not to employ the dimer and/or its esters, but to employ the higher oligomers.

In such a case, these compounds may be regarded as short chain polyethylene glycols, with a high proportion of long side chains, some of which are inert and some of which contain an acid or ester group as a functional moiety.

It has now been found that the above object can be fulfilled by a composition comprising a compound (A): 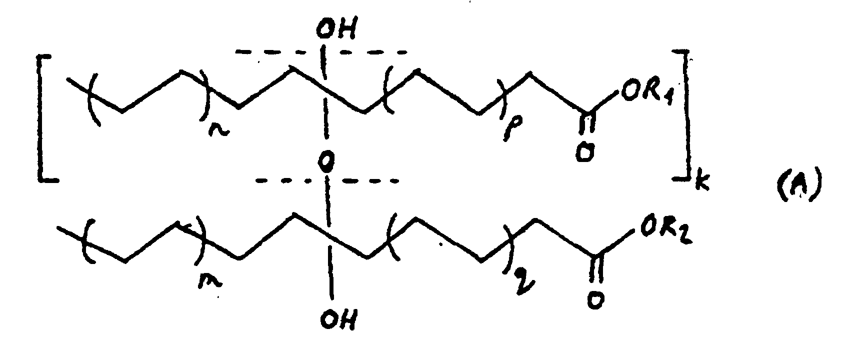 wherein
each R₁ and R₂ in each repeat unit are independently hydrogen, an alkyl group or an alkenyl group,
k is a number between 1 and 3,
m and q and each n and p in each repeat unit are independently a number between 1 and 5, and
each hydroxylic or ether 0 atom is bonded to either of the two adjacent carbon atoms to which its bond is directed with the proviso that that carbon atom only bears one such 0 atom, and
with the provisos that
the total number of carbon atoms in compound (A) is even and between 12(k + 1) and 22(k + 1), and
either
where k is 1, the composition comprises at least 35% by weight of the corresponding compound (A), or
where k is > 1, the composition comprises at least 15% by weight of a compound (A).

Preferably, in the above R₁ and R₂ are methyl or ethyl groups.

Depending on the area of application, it may be preferred however that the composition as set out above comprises at least 60%, or even 90%, by weight of compound (A), where k is 1, and where k is > 1, the composition comprises at least 25%, or even 35%, by weight of a compound (A).

Preferably, depending on the available choice of starting materials, in compound (A), m, n, p and q are independently 2 or 3.

In one aspect, the present invention also fulfils the above object by providing a composition comprising at least 35% by weight of a compound (I) and/or compound (II) and/or compound (III): 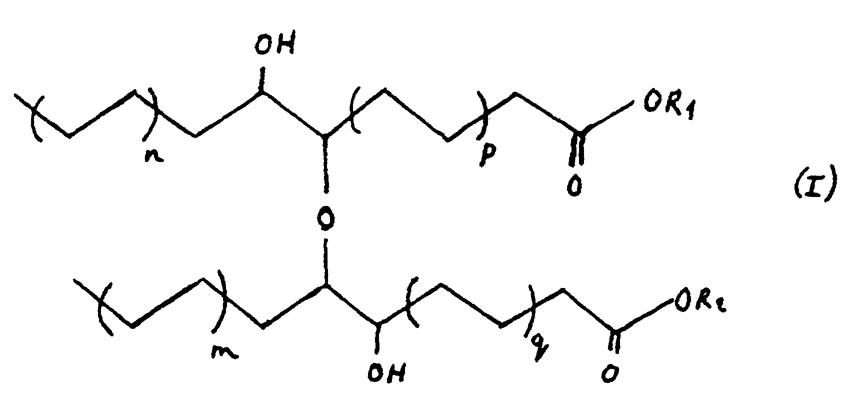 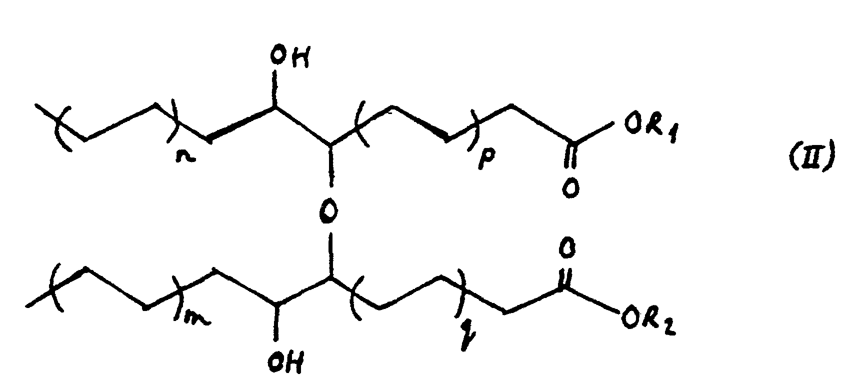 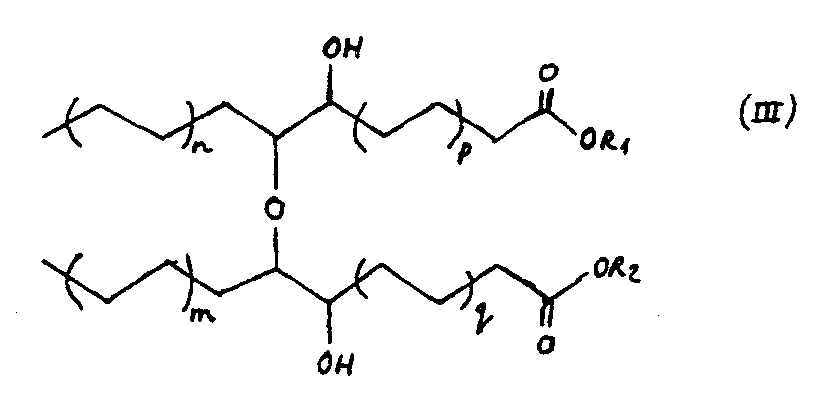 wherein
R₁ and R₂ are independently hydrogen, an alkyl group or an alkenyl group, and
m, n, p and q are independently a number between 1 and 5, with the proviso that the total number of carbon atoms in each compound (I), (II) and/or (III) is even and between 24 and 44.

Preferably, in the above R₁ and R₂ are methyl or ethyl groups.

Depending on the area of application, it may be preferred however that the composition as set out above comprises at least 60%, or even 90% by weight of compound (I), (II) and/or (III).

Preferably, depending on the available choice of starting materials, in compound (I) and/or compound (II) and/or compound (III) m, n, p and q are independently 2 or 3.

The present invention also embraces a composition comprising at least 35% by weight of a compound (IV) and/or compound (V) and/or compound (VI)

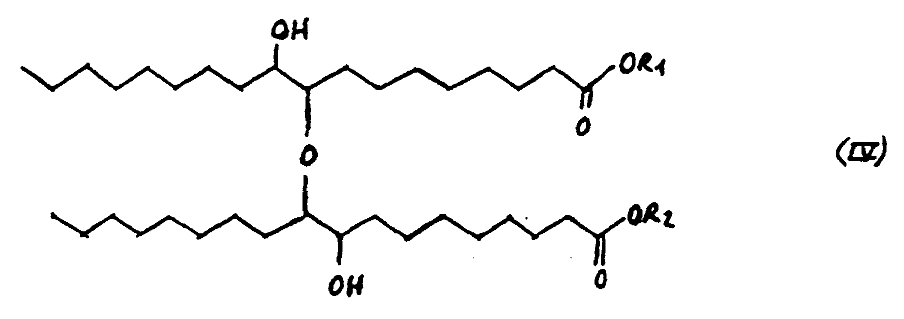

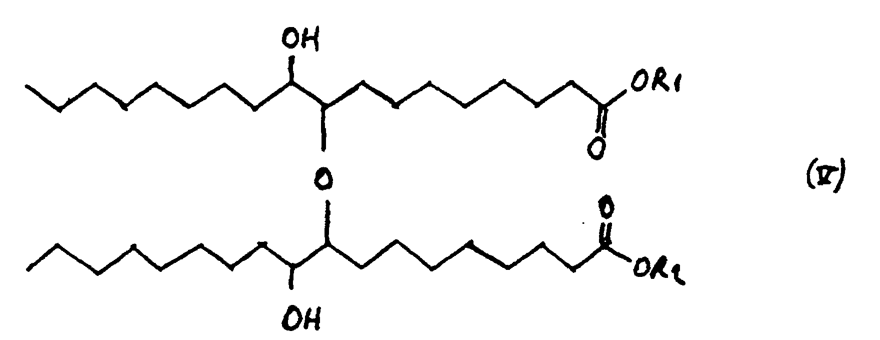

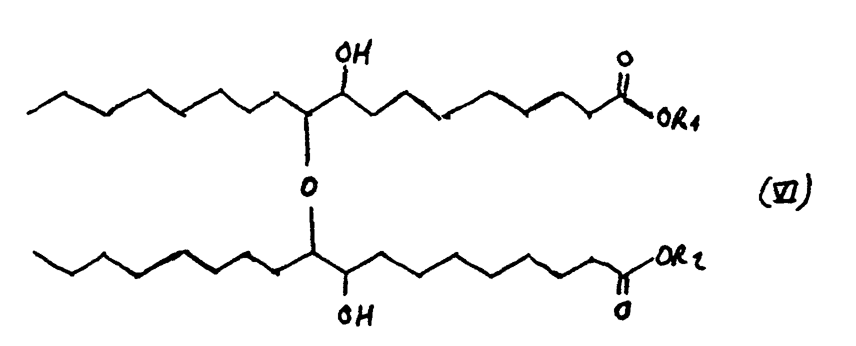

In compounds (IV), (V) and (VI), R₁ and R₂ are independently hydrogen, an alkyl group or an alkenyl group.

As above, depending on the area of application, it may be preferred however that the composition of the present invention contains a higher proportion of the compounds (IV), (V) and/or (VI). Therefore, it may be preferred however that the composition as set out above comprises at least 60%, or even 90% by weight of composition comprising 35% by weight of compound (IV) and/or (V) and/or (VI).

The present invention also embraces a composition comprising at least 15% by weight of a compound (VII): 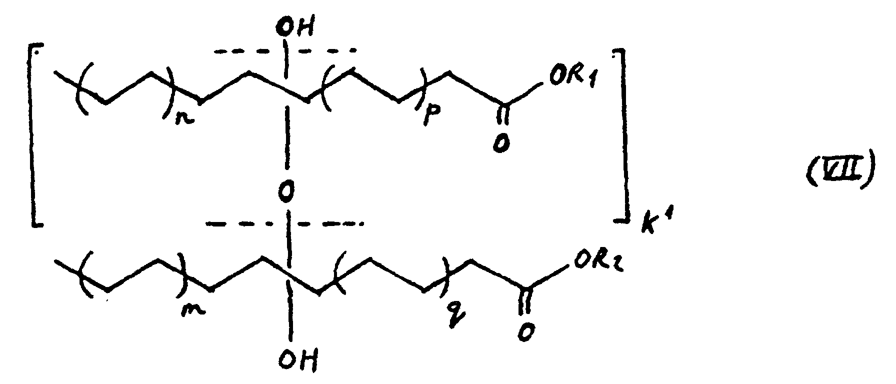 wherein
each R₁ and R₂ in each repeat unit are independently hydrogen, an alkyl group or an alkenyl group,
k¹ is 2 or 3,
m and q and each n and p in each repeat unit are independently a number between 1 and 5,
each hydroxylic or ether 0 atom is bonded to either of the two adjacent carbon atoms to which its bond is directed with the proviso that that carbon atom only bears one such 0 atom, and
with the provisos that the total number of carbon atoms in the compound (VII) is even and between 12(k¹ + 1) and 22(k¹ + 1).

Preferably, depending on the available choice of starting materials, in compound (VII), p and q are independently 2 or 3.

Preferably, the composition as set out above comprises at least 25%, or even 35% by weight of compound (VII).

It has been found that the compounds (A) can be obtained as side-products during acid-catalysed hydrolysis of fatty acid epoxides and/or the alkyl or alkenyl esters thereof. In general, when the esters are used as a starting material, a higher degree of compounds (A) are formed when compared with using the fatty acid epoxides themselves. Such an acid-catalysed hydrolysis of fatty acid epoxides and/or the esters thereof is a known process for manufacturing dihydroxy fatty acids (or triglycerides thereof) such as (vicinal) dihydroxystearic acid or dihydroxystearates. A process like this is described in DE 4125031 and by S. Dahlke et al, JAOCS 72(3), 349-353, 1995.

The fatty acid epoxides may be produced by epoxidation of unsaturated fatty acids (such as oleic acid) with a peroxide. The same holds for esters of fatty acid epoxides, e.g. triglycerides.

Thus, dihydroxystearic acid can be prepared from the epoxide made from so treating a source containing oleic acid (which is widely available) and the side-products obtainable include compound (IV) and/or compound (V) and/or compound (VI).

Therefore, the present invention also provides a process for manufacturing a composition according to the present invention, wherein a mixture comprising a compound (A) in which each R₁ and R₂ in each repeat unit are independently an alkyl group or an alkenyl group, is subjected to a distillation for separating the mixture into a first fraction rich in dihydroxy fatty acids (or esters thereof) and a second fraction which is a composition of the present invention, and then as desired R₁ and/or R₂ are converted to hydrogen.

Said mixture is obtainable by acid-catalysed hydrolysis of fatty acid epoxides and/or the alkyl or alkenyl esters thereof.

In one embodiment of this process, the present invention also provides a process for manufacturing a composition according to the present invention, wherein a mixture comprising any of the compounds (I) to (III) in which each R₁ and R₂ is independently an alkyl group or an alkenyl group, is subjected to a distillation for separating the mixture into a first fraction rich in dihydroxy fatty acids (or esters thereof) and a second fraction which is a composition of the present invention, and then as desired R₁ and/or R₂ are converted to hydrogen.

In another embodiment of this process, the present invention also provides a process for manufacturing a composition according to the present invention, wherein a mixture comprising any of the compounds (IV) to (VI) in which each R₁ and R₂ is independently an alkyl group or an alkenyl group, is subjected to a distillation for separating the mixture into a first fraction rich in dihydroxystearic acid (or esters thereof) and a second fraction which is a composition of the present invention, and then as desired R₁ and/or R₂ are converted to hydrogen.

In a third embodiment of this process, the present invention also provides a process for manufacturing a composition according to the present invention, wherein a mixture comprising a compound (VII) in which each R₁ and R₂ in each repeat unit are independently an alkyl group or an alkenyl group, is subjected to a distillation for separating the mixture into a first fraction rich in dihydroxy fatty acids (or esters thereof) and a second fraction which is a composition of the present invention, and then as desired R₁ and/or R₂ are converted to hydrogen.

For obtaining the compounds (I) to (III) in a purer form, the second fraction is subjected to a second distillation for removing higher boiling substances from the composition of the present invention.

By doing so, trimers and tetramers (VII) and higher oligomers, similar in structure to the ether dimers (I) to (III) can be removed from the dimers.

Equally, the same second distillation may be used for removing the compounds (I) to (III) to obtain the trimers and tetramers (VII) in a purer form.

Preferably, all said distillations are carried out using molecular distillation.

If it is desired that R₁ and/or R₂ are converted to hydrogen, such conversion will be effected after all desired distillations have been carried out.

The esters in the final product composition of the present invention may be converted to the corresponding fatty acid derivatives by conventional saponification by base-catalysed hydrolysis or alcoholysis with conventional removal of the by-product alcohol, followed by neutralisation, or by enzymolysis.

In the case where fatty acid epoxide esters are used as a starting material, the alcohol part of the esters is preferably an alkyl or alkenyl alcohol with 1-6 carbon atoms.

Most preferred alcohol residues for this purpose are those of methanol and ethanol.

For some purposes, it may be preferred not to employ the dimer esters (I) to (III), but to employ the higher oligomers.

In such a case, these compounds may be regarded as short chain polyethylene glycols, with a high proportion of long side chains, some of which are inert and some of which contain an acid or ester group as a functional moiety.

The compositions according to the present invention may be used as such, or may subsequently be further processed, depending on the intended use.

These highly functional dimers are especially useful in polyamides, polyurethanes and polyesters, water-based paints and coatings, inks, etc.

The compositions and the process for the manufacture thereof are further exemplified by the following non-limiting examples.

### Example 1a

A mixture of dihydroxy fatty acid methyl esters was obtained by acid-catalysed hydrolysis of an epoxidised methyl oleate feedstock.

This consisted of
approximately 50% methyl 9,10-dihydroxystearate,
approximately 25% dimethyl esters of dihydroxy fatty acid ether dimers (primarily methyl 9,10-dihydroxystearate ether dimers),
approximately 5% polymethyl esters of dihydroxy fatty acid ether polymers (primarily methyl 9,10-dihydroxystearate ether polymers),
approximately 6% dihydroxy derivatives of methyl linoleate, and
approximately 14% methyl esters of non-hydroxylated fatty acids (including methyl palmitate and methyl stearate), made as described below in lines 10 - 12 of page 16.
Analysis was carried out by GLC/GPC.

23.9 kg of this mixture (OH value 228) was subjected to a molecular distillation at 200°C and 0.001 mbar, resulting in two fractions A and B of respectively 16.6 kg and 7,2 kg. Fractions A and B respectively were characterised by OH values 264 and 151, indicating that hardly any loss of hydroxyl functionality had occurred during distillation.

Fraction A consisted of a mixture of monomeric fatty acids and esters, being
approximately 67% methyl dihydroxystearate,
approximately 9% dihydroxy derivatives of methyl linoleate, and
approximately 27% methyl esters of non-hydroxylated fatty acids (including methyl palmitate and methyl stearate).

Fraction B contained polymeric material, consisting of a mixture of
approximately 72% dimethyl esters of dihydroxy fatty acid ether dimers and
approximately 28% dihydroxy ether polymers (primarily trimer).

2.26 kg of fraction B was subjected to a second molecular distillation at 270°C and 0.003 mbar, resulting in a top fraction of 1.58 kg of 90% of ether dimers and 0.58 kg of distillation bottom containing dihydroxy ether polymers.

Top and bottom fractions respectively had OH values of 156 and 130 again indicating that molecular distillation hardly affects the hydroxyl functionality.

### Example 1b

191.8 g of the 90% methyl esters of dihydroxy fatty acid ether dimers was reacted with 43.5 g 90% hexamethylenediamine and 18.9 g oleic acid.

After reaction at atmospheric pressure for 3 hr at 150°C and another 3 hr at 200°C, amine values changed from 152 to 4.7 and acid values from 15 to 4.4. indicating a conversion of approximately 97%.

Gel permeation chromatography showed that a polyamide was formed with an average molecular weight of 7300 g/mole. The polyamide had a viscosity of 570 cP at 160°C.

### Example 2

228.3 g of the 90 % methyl esters of dihydroxy fatty acid ether dimers was reacted with 21.7 g ethylenediamine. After reaction at atmospheric pressure for 7 hr at maximum 200°C, an amine value of 11.5 mg KOH/g was obtained indicating a conversion of approximately 93 %.

Gel permeation chromatography showed that a polyamide was formed with an average molecular weight of 5400 g/mole. The polyamide had a viscosity of 465 cP at 160°C.

### Example 3

209.0 g of 70 % methyl esters of dihydroxy fatty acid ether dimers (prepared as in Example 1a) and 20.0 g oleic acid was reacted with 21.0 g ethylenediamine. After reaction at atmospheric pressure for 7 hr at maximum 200°C, an amine value of 3.6 mg KOH/g and an acid value of 4.1 mg KOH/g was obtained.

Gel permeation chromatography showed that a polyamide was formed with an average molecular weight of 8100 g/mole. The polyamide had a viscosity of 800 cP at 160°C.

### Example 4a

300 g of the 90 % methyl esters of dihydroxy fatty acid ether dimers (prepared as in Example 1a) was mixed with a solution of 131 g potassium hydroxide (85%) in 135 ml water + 270 g ethanol (96%) and heated to 82°C under reflux. This temperature was maintained for ca. 3 hours. Ethanol (and methanol) was then distilled off gradually. In ca. 2.5 hours, a total of 270 g distillate was collected, while the boiling temperature was raised to 92°C. 50 ml of water was added at the end of the distillation to compensate for the syrup-like behaviour of the potassium soaps. The temperature was decreased to 60°C.

141 g sulphuric acid (95-98%) in 405 ml water was added in small portions at a time in order to set free the organic acids from the potassium soaps. During the first part of the neutralisation, external water cooling was applied in order to remove some of the reaction heat.

After addition of all the sulphuric acid, continuous stirring was applied during one hour.

After settling of the water phase, this phase was removed; also some solid precipitated crystal material was removed.

Finally the product was washed 4 times with portions of 300 g water and dried during one hour at 90 °C and 20 mbar pressure. The product had an acid value of 182 mg KOH/g and a saponification value of 183 mg KOH/g.

### Example 4b

200.5 g of the above mentioned product was reacted with 19.5 g ethylenediamine. After reaction at atmospheric pressure for 7 hr at maximum 200°C an amine value of 7.2 mg KOH/g and an acid value of 6.5 mg KOH/g was obtained. Gel permeation chromatography showed that a polyamide was formed with an average molecular weight of 6600 g/mole. The polyamide had a viscosity of 1100 cP at 160°C.

### Example 5a

234.0 g of a mixture of dihydroxy fatty acid methyl esters consisting of
approximately 50% methyl 9,10-dihydroxystearate,
approximately 25% dimethyl esters of dihydroxy fatty acid ether dimers,
approximately 5 % polymethyl esters of dihydroxy fatty acid ether polymers,
approximately 6 % dihydroxy derivatives of methyl linoleate, and
approximately 14% methyl esters of non-hydroxylated fatty acids
was prepared using an acid-catalysed hydrolysis of fatty acid ester epoxides like that described in DE 4125031 and by S. Dahlke et al, JAOCS 72(3), 349-353, 1995.

### Example 5b

234.0 g of the mixture was reacted with 22.3 g ethylenediamine. After reaction at atmospheric pressure for 7 hr at maximum 200°C an amine value of 5.0 mg KOH/g was obtained.

Gel permeation chromatography showed that a product was formed with an average molecular weight of 1850 g/mole. The product had a viscosity of 39.5 cP at 160°C.

It will be seen that known mixtures comprising fatty acid ether diners are not useful as building blocks in subsequent further processing for use for polymers, paints, etc.

## Claims

1. A composition comprising a compound (A): 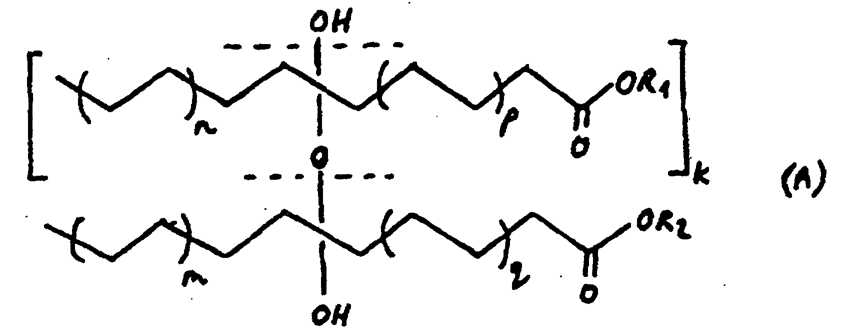 wherein
each R₁ and R₂ in each repeat unit are independently hydrogen, an alkyl group or an alkenyl group,
k is a number between 1 and 3,
m and q and each n and p in each repeat unit are independently a number between 1 and 5, and
each hydroxylic or ether 0 atom is bonded to either of the two adjacent carbon atoms to which its bond is directed with the proviso that that carbon atom only bears one such 0 atom, and
with the provisos that
the total number of carbon atoms in compound (A) is even and between 12(k + 1) and 22(k + 1), and
either
where k is 1, the composition comprises at least 35% by weight of the corresponding compound (A), or
where k is > 1, the composition comprises at least 15% by weight of a compound (A).

2. A composition according to claim 1, comprising at least 35% by weight of a compound (I) and/or compound (II) and/or compound (III) 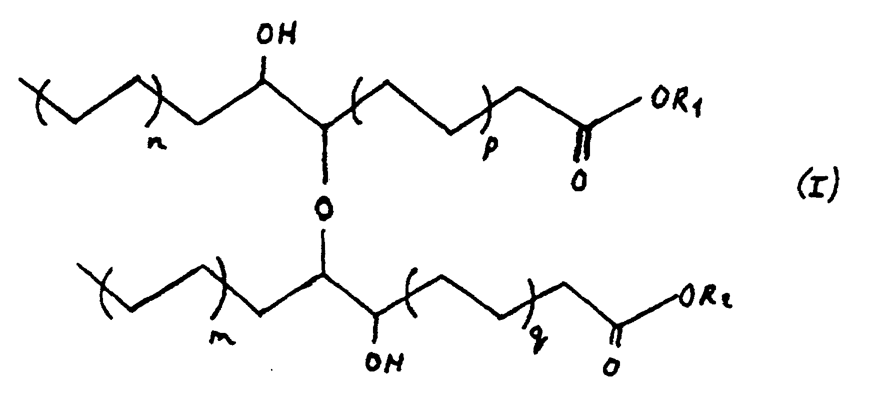 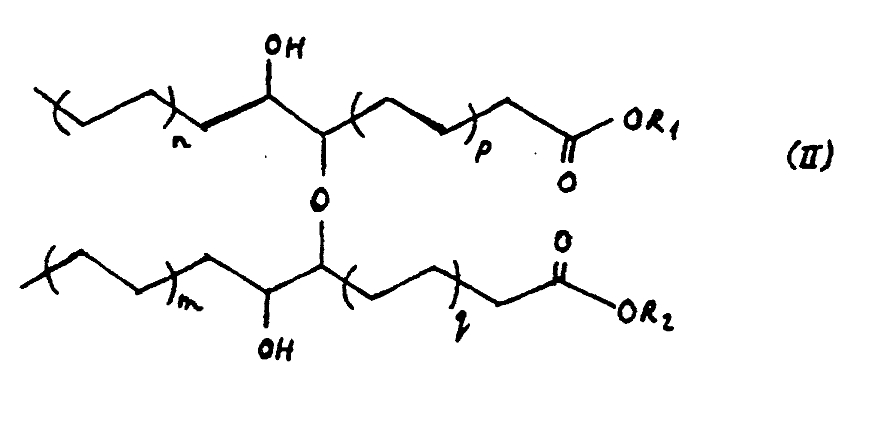 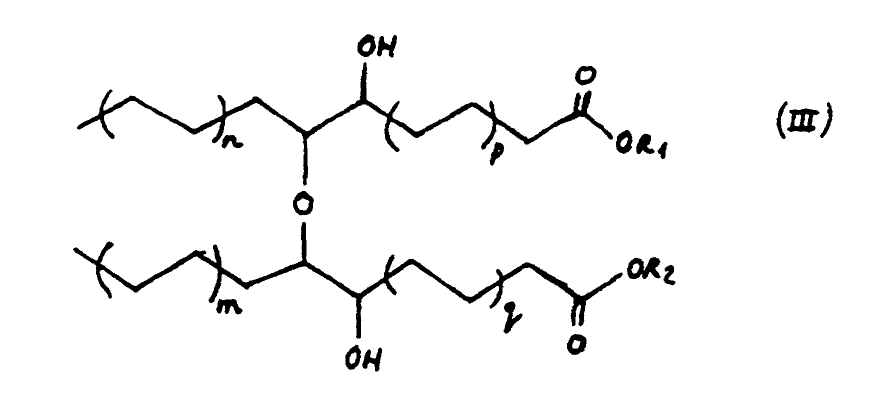 wherein R₁ and R₂ are independently hydrogen, an alkyl group or an alkenyl group, and m, n, p and q are independently a number between 1 and 5, with the proviso that the total number of carbon atoms in each compound (I), (II) and/or (III) is even and between 24 and 44.

3. A composition according to claim 1, **characterised in that** it comprises at least 60% by weight of a compound (I) and/or (II) and/or (III).

4. A composition according to claim 1, comprising at least 35% by weight of compound (IV) and/or compound (V) and/or (VI). 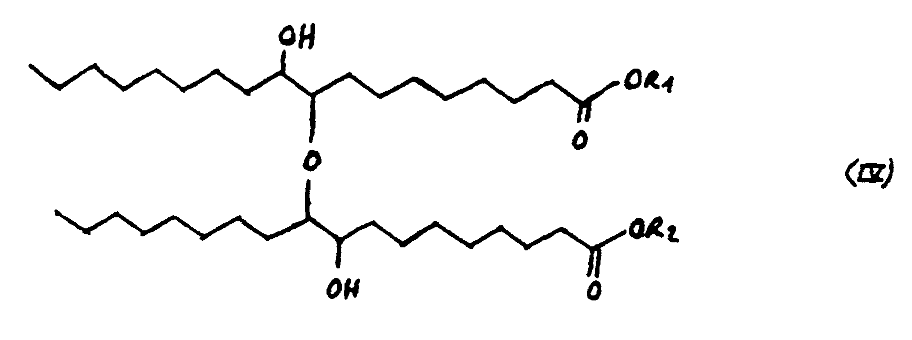 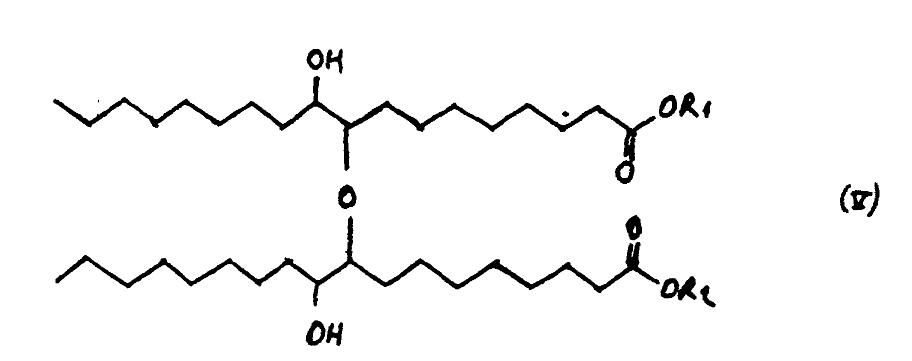 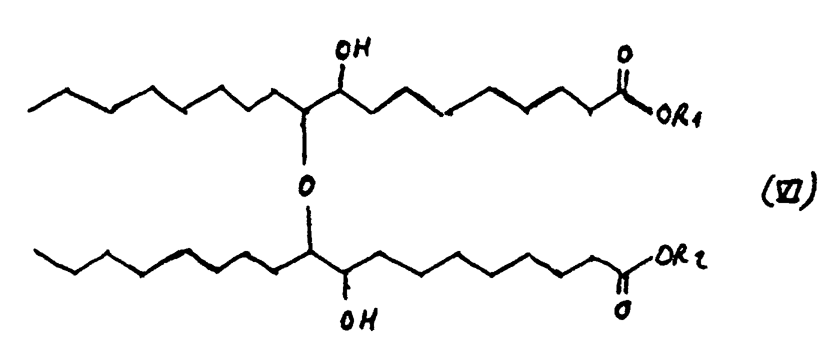 wherein R₁ and R₂ are independently hydrogen, an alkyl group or an alkenyl group.

5. A composition according to claim 4, **characterised in that** it comprises at least 60% by weight of a compound (IV) and/or (V) and/or (VI).

6. A composition according to claim 1, comprising at least 15% by weight of a compound (VII): 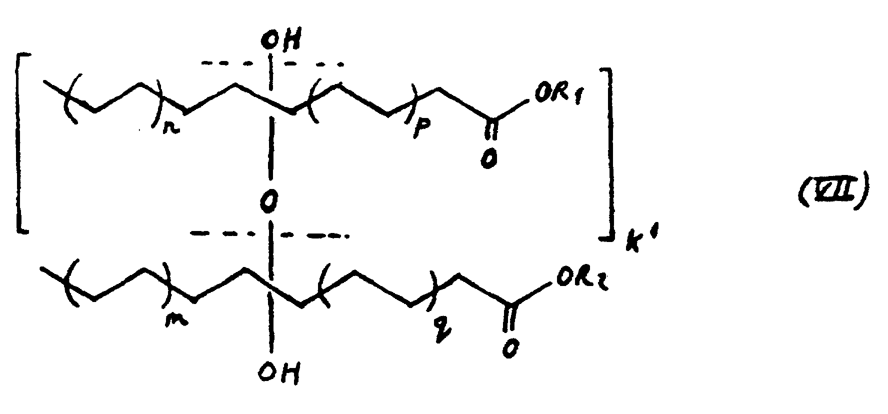 wherein
each R₁ and R₂ in each repeat unit are independently hydrogen, an alkyl group or an alkenyl group,
k¹ is 2 or 3,
m and q and each n and p in each repeat unit are independently a number between 1 and 5,
each hydroxylic or ether 0 atom is bonded to either of the two adjacent carbon atoms to which its bond is directed with the proviso that that carbon atom only bears one such 0 atom, and
with the provisos that the total number of carbon atoms in the compound (VII) is even and between 12(k¹ + 1) and 22(k¹ + 1).

7. A process for manufacturing a composition according to claim 1, wherein a mixture comprising the compound (A) in which each R₁ and R₂ in each repeat unit are independently an alkyl group or an alkenyl group, is subjected to a distillation for separating the mixture into a first fraction rich in dihydroxy fatty acids (or esters thereof) and a second fraction rich in the compound (A), and then as desired R₁ and/or R₂ are converted to hydrogen.

8. A process for manufacturing a composition according to claim 2, wherein a mixture comprising any of the compounds (I) to (III) in which each R₁ and R₂ are independently an alkyl group or an alkenyl group, is subjected to a distillation for separating the mixture into a first fraction rich in dihydroxy fatty acids (or esters thereof) and a second fraction rich in any of the compounds (I) to (III), and then as desired R₁ and/or R₂ are converted to hydrogen.

## Patentansprüche

1. Zusammensetzung, die eine Verbindung (A) umfasst: 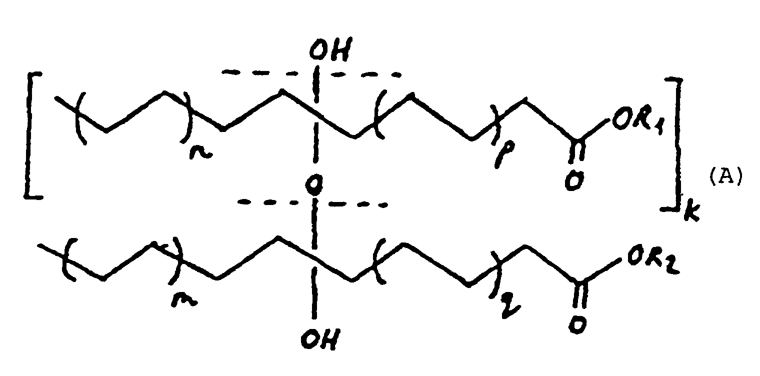 worin
jedes R₁ und R₂ in jeder Wiederholungseinheit unabhängig Wasserstoff, eine Alkyl- oder Alkenylgruppe ist,
k eine Zahl zwischen 1 und 3 ist,
m und q und jedes n und p in jeder Wiederholungseinheit unabhängig eine Zahl zwischen 1 und 5 sind, und
jedes hydroxylische oder Ether-O-Atom an jedes der beiden angrenzenden Kohlenstoffatome, an welche es gebunden ist, unter der Bedingung gerichtet ist,
dass das Kohlenstoffatom nur ein derartiges O-Atom trägt, und
unter den Bedingungen, dass
die Gesamtzahl an Kohlenstoffatomen in Verbindung (A) geradzahlig und zwischen 12(k+1) und 22(k+1) ist, und
entweder
wenn k gleich 1 ist, die Zusammensetzung wenigstens 35 Masseprozent der entsprechenden Verbindung (A) umfasst, oder,
wenn k > 1 ist, die Zusammensetzung wenigstens 15 Masseprozent einer Verbindung (A) umfasst.

2. Zusammensetzung nach Anspruch 1, die wenigsten 35 Masseprozent einer Verbindung (I) und/oder Verbindung (II) und/oder Verbindung (III) umfasst: 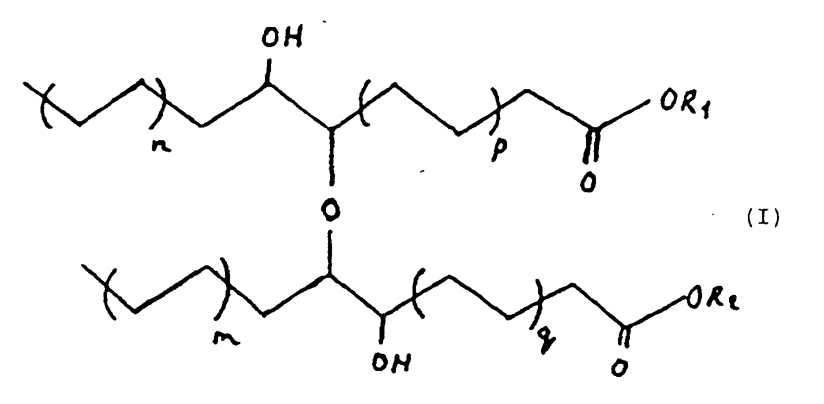 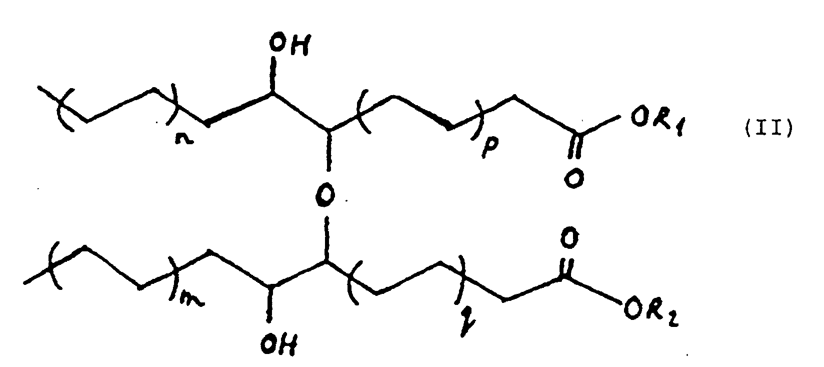 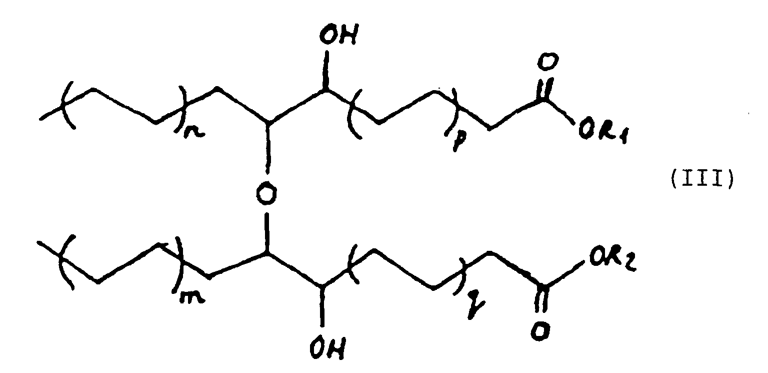 worin
R₁ und R₂ unabhängig Wasserstoff, eine Alkyl- oder Alkenylgruppe sind, und m, n, p und q unabhängig eine Zahl zwischen 1 und 5 sind, unter der Bedingung, dass die Gesamtzahl der Kohlenstoffatome in jeder Verbindung (I), (II) und/oder (III) geradzahlig und zwischen 24 und 44 ist.

3. Zusammensetzung nach Anspruch 1, **dadurch gekennzeichnet, dass** sie wenigstens 60 Masseprozent einer Verbindung (I) und/oder (II) und/oder (III) umfasst.

4. Zusammensetzung nach Anspruch 1, **dadurch gekennzeichnet, dass** sie wenigstens 35 Masseprozent einer Verbindung (IV) und/oder (V) und/oder (VI) umfasst: 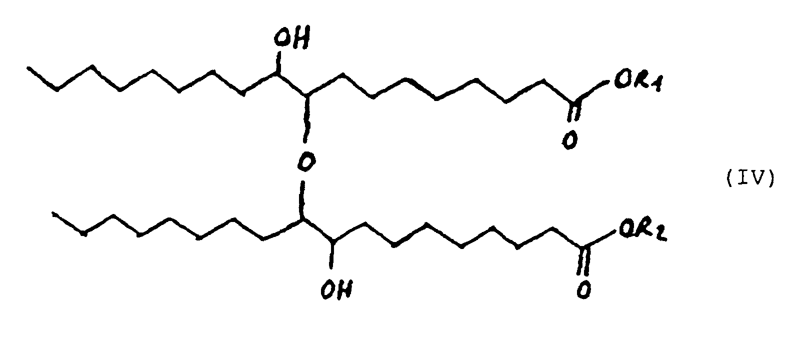 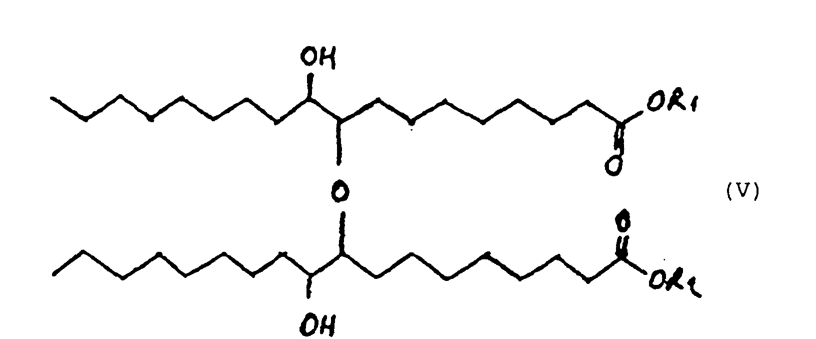 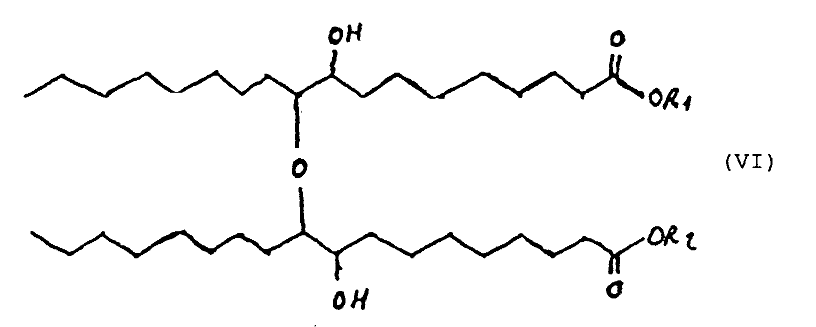 worin R₁ und R₂ unabhängig Wasserstoff, eine Alkyloder Alkenylgruppe sind.

5. Zusammensetzung nach Anspruch 4, **dadurch gekennzeichnet, dass** sie wenigstens 60 Masseprozent einer Verbindungen (IV) und/oder (V) und/oder (VI) umfasst.

6. Zusammensetzung nach Anspruch 1, **dadurch gekennzeichnet, dass** sie wenigstens 15 Masseprozent einer Verbindung (VII) umfasst. 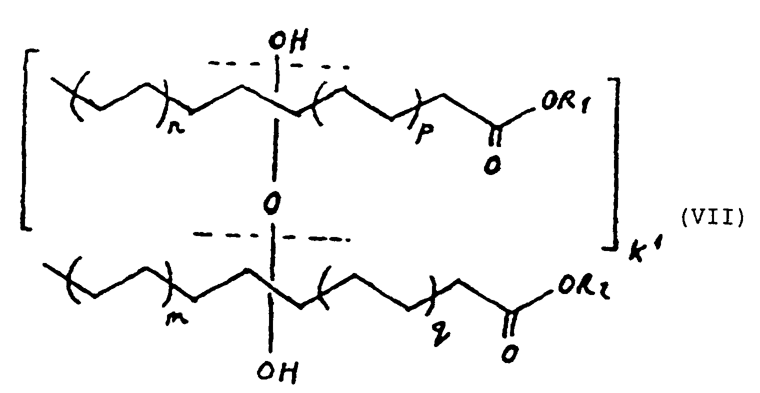 worin
jedes R₁ und R₂ in jeder Wiederholungseinheit unabhängig Wasserstoff, eine Alkyl- oder Alkenylgruppe ist,
k¹ gleich 2 oder 3 ist,
m und q und jedes n und p in jeder Wiederholungseinheit unabhängig eine Zahl zwischen 1 und 5 sind,
jedes hydroxylische oder Ether-O-Atom an jedes der beiden angrenzenden Kohlenstoffatome, an welche es gebunden ist, unter der Bedingung gerichtet ist, dass das Kohlenstoffatom nur ein derartiges O-Atom trägt, und
unter den Bedingungen, dass die Gesamtzahl an Kohlenstoffatomen in Verbindung (VII) geradzahlig und zwischen 12(k¹+1) und 22(k¹+1) ist.

7. Verfahren zur Herstellung einer Zusammensetzung nach Anspruch 1, worin eine Mischung, die die Verbindung (A) umfasst, in welcher jedes R₁ und R₂ in jeder Wiederholungseinheit unabhängig eine Alkyl- oder Alkenylgruppe sind, einer Destillation zur Trennung der Mischung in eine an Dihydroxyfettsäuren (oder deren Estern) reiche erste Fraktion und eine zweite Fraktion, welche reich an der Verbindung (A) ist, unterzogen wird und dann, falls erwünscht, R₁ und/oder R₂ in Wasserstoff umgewandelt werden.

8. Verfahren zur Herstellung einer Zusammensetzung nach Anspruch 2, worin eine Mischung, die die Verbindungen (I) bis (III) umfasst, in welchen jedes R₁ und R₂ unabhängig eine Alkyl- oder Alkenylgruppe sind, einer Destillation zur Trennung der Mischung in eine an Dihydroxyfettsäuren (oder deren Estern) reiche erste Fraktion und eine zweite Fraktion, welche reich an den Verbindungen (I) bis (III) ist, unterzogen wird und dann falls erwünscht R₁ und/oder R₂ in Wasserstoff umgewandelt werden.

## Revendications

1. Composition comprenant un composé (A) : 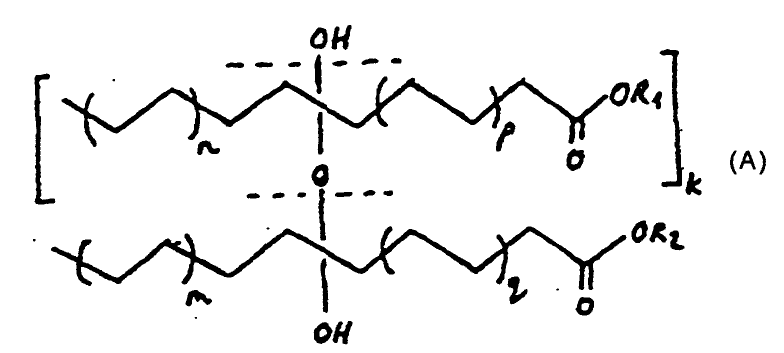 dans lequel :
chaque R₁ et R₂ dans chaque unité répétée sont indépendamment un atome d'hydrogène, un groupe alkyle ou un groupe alcényle,
k est un nombre compris entre 1 et 3,
m et q et chaque n et p dans chaque unité répétée sont indépendamment un nombre compris entre 1 à 5, et
chaque atome d'oxygène de groupe hydroxy ou de groupe éther est relié à l'un ou l'autre de deux atomes de carbone adjacents vers lesquels sa liaison est dirigée à condition que cet atome de carbone porte seulement un tel atome d'oxygène, et
à condition que le nombre total d'atomes de carbone dans le composé (A) soit pair et compris entre 12(k+1) et 22(k+1) et que, soit lorsque k est 1, la composition comprenne au moins 35% en poids du composé correspondant (A),
soit lorsque k est supérieur à 1, la composition comprenne au moins 15% en poids d'un composé (A).

2. Composition suivant la revendication 1, comprenant au moins 35% en poids d'un composé (I) et/ou d'un composé (II) et/ou d'un composé (III) 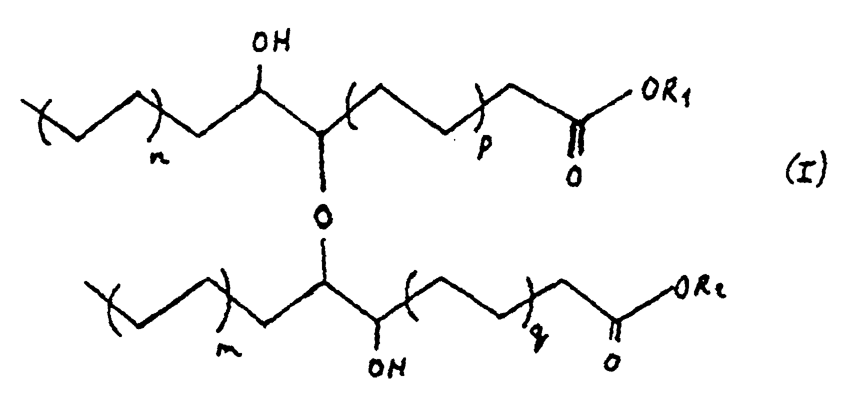 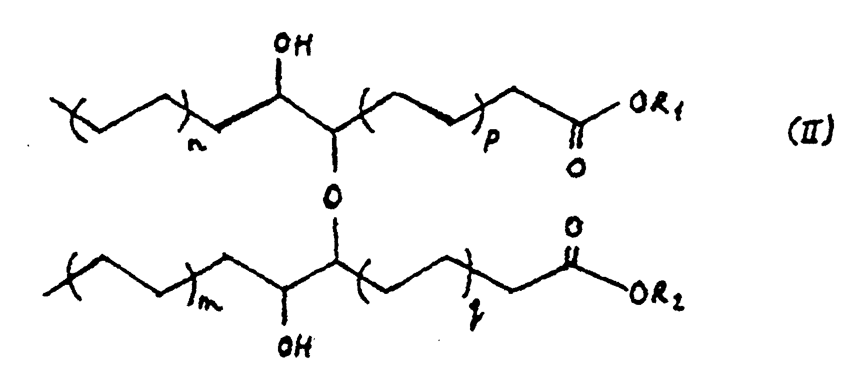 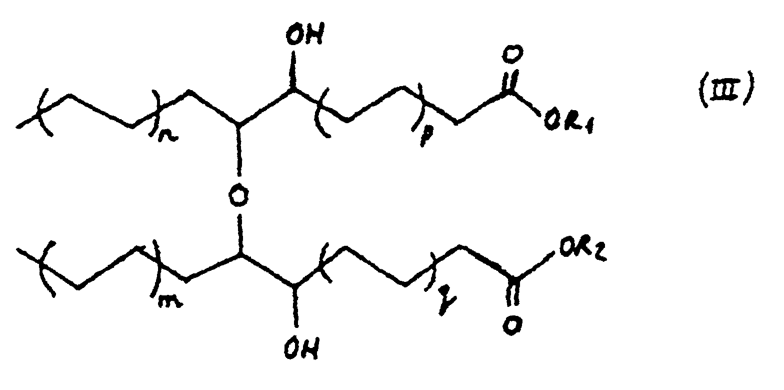 dans lesquels :
R₁ et R₂ sont indépendamment un atome d'hydrogène, un groupe alkyle ou un groupe alcényle, et
m, n, p et q sont indépendamment un nombre compris entre 1 et 5, à condition que le nombre total d'atomes de carbone dans chaque composé (I), (II) et/ou (III) soit pair et compris entre 24 et 44.

3. Composition suivant la revendication 1, **caractérisée en ce qu'**elle comprend au moins 60% en poids d'un composé (I), et/ou (II) et/ou (III).

4. Composition suivant la revendication 1,
comprenant au moins 35% en poids d'un composé (IV) et/ou d'un composé (V) et/ou d'un composé (VI) : 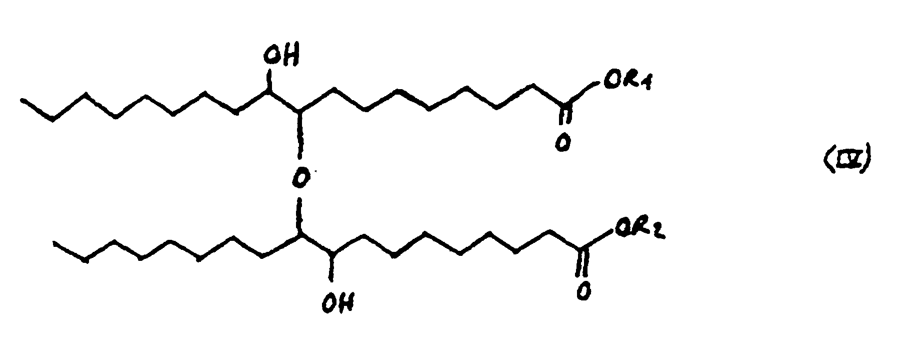 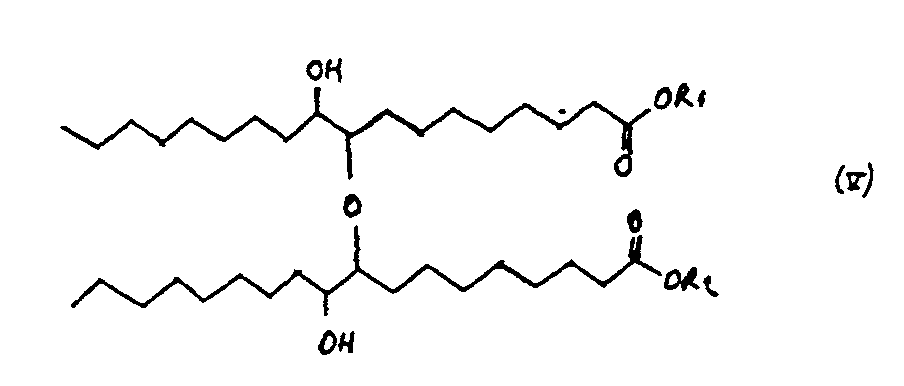 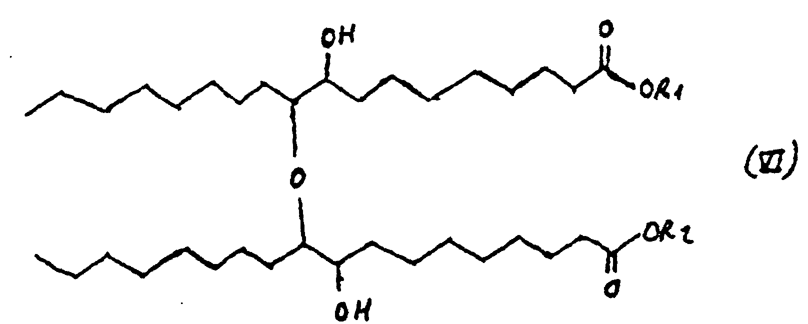 dans lesquels R₁ et R₂ sont indépendamment un atome d'hydrogène, un groupe alkyle ou un groupe alcényle.

5. Composition suivant la revendication 4, **caractérisée en ce qu'**elle comprend au moins 60% en poids d'un composé (IV) et/ou d'un composé (V) et/ou d'un composé (VI).

6. Composition suivant la revendication 1, comprenant au moins 15% en poids d'un composé (VII) : 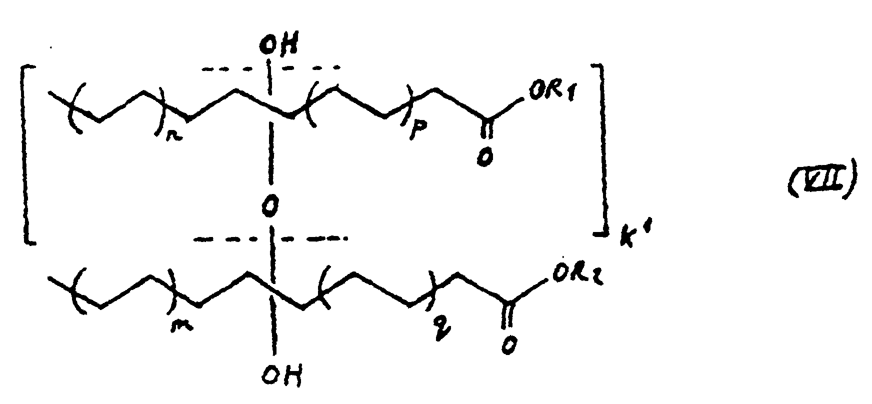 dans lequel :
chaque R₁ et R₂ dans chaque unité répétée sont indépendamment un atome d'hydrogène, un groupe alkyle ou un groupe alcényle,
k¹ est 2 ou 3,
m et q et chaque n et p dans chaque unité répétée sont indépendamment un nombre compris entre 1 à 5,
chaque atome d'oxygène de groupe hydroxy ou de groupe éther est relié à l'un ou l'autre de deux atomes de carbone adjacents vers lesquels sa liaison est dirigée à condition que cet atome de carbone porte seulement un tel atome d'oxygène, et
à condition que le nombre total d'atomes de carbone dans le composé (VII) soit pair et compris entre 12(k¹+1) et 22(k¹+1).

7. Procédé pour la fabrication d'une composition suivant la revendication 1, dans lequel un mélange comprenant un composé (A) dans lequel chaque R₁ et R₂ dans chaque unité répétée sont indépendamment un groupe alkyle ou un groupe alcényle, est soumis à une distillation pour séparer le mélange en une première fraction riche en acides dihydroxy gras (ou leurs esters) et une seconde fraction riche en composé (A) et ensuite, en fonction de ce qui est désiré, R₁ et/ou R₂ sont convertis en atome d'hydrogène.

8. Procédé pour la fabrication d'une composition suivant la revendication 2, dans lequel un mélange comprenant l'un quelconque des composés (I) à (III), dans lesquels chaque R₁ et R₂ sont indépendamment un groupe alkyle ou un groupe alcényle, est soumis à une distillation pour séparer le mélange en une première fraction riche en acides dihydroxy gras (ou leurs esters) et une seconde fraction riche en l'un quelconque des composés (I) à (III), et ensuite selon ce qui est désiré, R₁ et/ou R₂ sont convertis en atome d'hydrogène.
